# EUROPEAN PATENT APPLICATION

(11) **EP 2 149 547 A1**
(43) Date of publication of application: **03.02.2010**
(21) Application number: 08161441.4
(22) Date of filing: 30.07.2008
(51) Int. Cl.: C07C 57/60, C07C 69/65, C07D 205/08, C07D 263/26

(54) **Process for the synthesis of ezetimibe and intermediates useful therefor**

(71) Applicant: LEK Pharmaceuticals d.d., 1526 Ljubljana (SI)
(72) Inventor: The designation of the inventor has not yet been filed
(74) Representative: Opresnik, Marko

(57) **Abstract**

The present invention discloses novel and useful intermediates for the synthesis of ezetimibe (EZT), which intermediates share a characteristic *Z*-isomeric structure. Based on *Z*-5-(4-fluorophenyl)-pent-4-enoic acid, and proceeding the synthesis through further *Z-*intermediates, a total synthesis is presented to obtained final ezetimibe in high yields.

## Description

### Field of the invention

The present invention relates to a process for the synthesis of production of ezetimibe (in the following sometimes abbreviated as EZT) and for the preparation of a pharmaceutical composition containing the same, as well as to novel intermediates useful therefor.

### Background of the invention

Ezetimibe (EZT), chemically defined as (3R, 4S)-1-(p-fluorophenyl)-type-((3S)-3-(p-fluorophenyl)-3-hydroxypropyl)-4-(p-hydroxyphenyl)-2-azetidinone, is known as a useful drug, in particular as an anti-cholesteremic agent, as an anti-hyperlipidemic agent and notably an intestinal cholesterol absorption inhibitor, as an anti-lipemic agent and as an antimetabolite.

Document US-A-5 856 473 discusses in the background section various conventional processes for preparing ezetimibe, and the document itself proposes new processes for the synthesis of ezetimibe. One synthetic route starts from *E*-5-(4-fluorophenyl)-pent-4-enoic acid and proceeds further via *E*-intermediates. Similar information is described by S.B. Rosenblum et al. in Tetrahedron 56, pp. 5735-5742 (2000). Further literature about the synthesis of *E*-5-(4-fluorophenyl)-pent-4-enoic acid is represented in the Journal of Organic Chemistry 31(1), pp. 147-153 (1966), Chemical Communications 2006, pp. 643-645, and Journal of Organometallic Chemistry 692, pp. 2270-2281 (2007).

The object of the present invention was to provide an improved process and new means for the synthesis or production of ezetimibe (EZT), which in particular enables to obtain a higher total yield.

### Summary of the invention

In a first aspect, the present invention provides novel and useful intermediates for the synthesis of ezetimibe (EZT), which intermediates share a characteristic *Z*-isomeric structure. According to the present invention, it was found that when starting with compounds based on Z-5-(4-fluorophenyl)-pent-4-enoic acid, and when proceeding the synthesis through *Z*-intermediates, the total yield for the synthesis of the final ezetimibe is remarkably increased. There is a surprising higher overall yield when starting and following the synthesis route through *Z*-precursors and *Z*-intermediates than through the respective *E*-analogues. In addition, Z-pent-4-enoic acid is prepared in significantly higher yield than E-pent-4-enoic acid by methods known in the literature.

Thus in a first aspect of the invention, there are provided the following *Z*-intermediate compounds as representative key compounds for the improved synthesis of ezetimibe according to the present invention.
(1) A compound with the following formula I 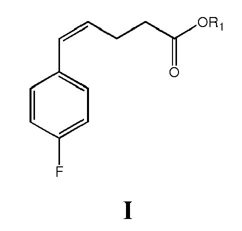 wherein R₁ is H or, respectively unsubstituted or substituted, alkyl, cycloalkyl, alkenyl, cycloalkenyl, alkynyl, aryl, aralkyl, arylcyloalkyl.
(2) The compound according to (1), wherein R₁ is H.
(3) A compound with the following formula II 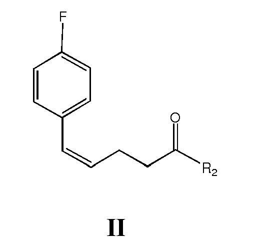 wherein R₂ is a leaving group.
   As used herein, the term "leaving group" is a chemical moiety which can be chemically cleaved from the carbonyl group, thereby eventually assisting in a subsequent step of performing a ring-cyclization step to form the characteristic azetidine-2-one structural moiety appearing in the ezetimibe structure. The leaving group preferably has an N-atom through which it is bonded to the carbonyl group of formula II. Further the leaving group preferably forms a ring structure, and/or may have a chiral atom in its chemical moiety.
(4) The compound according to (3) wherein R₂ is an N-heterocyclic group with the N-heteroatom forming amido group with carbonyl group in formula II, wherein preferably the N-heterocyclic group is alkylated or arylated, and more preferably is defined by a chiral S isomeric center.
(5) The compound according to (4) wherein R₂ is defined by the following formula III: 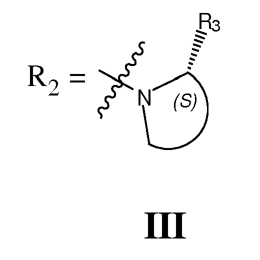
(6) A compound with the following formula IV 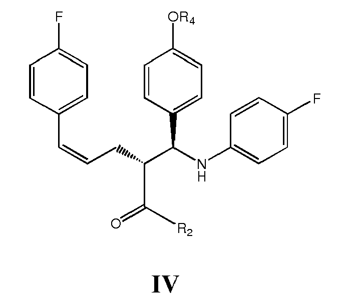 wherein R₂ is as defined in any one of (3) to (5), and R₄ is an OH-protecting group.
(7) A compound with the following formula V 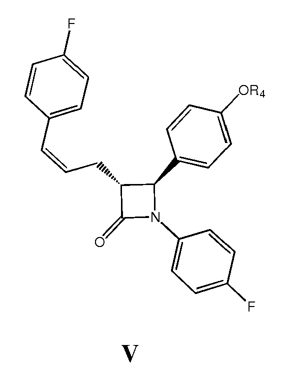 wherein R₄ is an OH-protecting group.
   According to another aspect of the present invention, there is provided:
(8) A process for producing 5-(4-fluorophenyl)pent-4-enoic acid or a derivative thereof, comprising subjecting 4-fluoro-benzaldehyde and a compound of formula VI to a Wittig reaction: 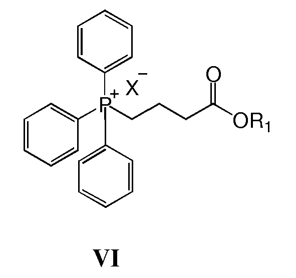 wherein X⁻ is a phosphonium salt anion, preferably a halogenide, and R₁ is as defined in (1) above.
(9) The process according to (8) wherein an (*E*,Z)-isomeric mixture of the obtained 5-(4-fluorophenyl)pent-4-enoic acid or derivatives thereof are isolated as the E-isomer and the Z-isomer, respectively.
(10) The process according to (8) or (9) wherein the isolated isomer is the Z isomer compound with the following formula I 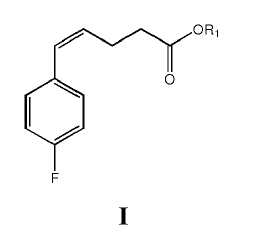 wherein R₁ is as defined in (1) above.
(11) The process according to any one of (8) to (10) wherein an ester derivative is used as a compound of formula VI, and after the Wittig reaction, the obtained ester group is subjected to saponification to give the corresponding 5-(4-fluorophenyl)-pent-4-enoic acid.
   According to another aspect of the present invention, there is provided:
(12) A process for producing a compound with formula II by a Wittig reaction of 4-fluoro-benzaldehyde with a compound of formula VII 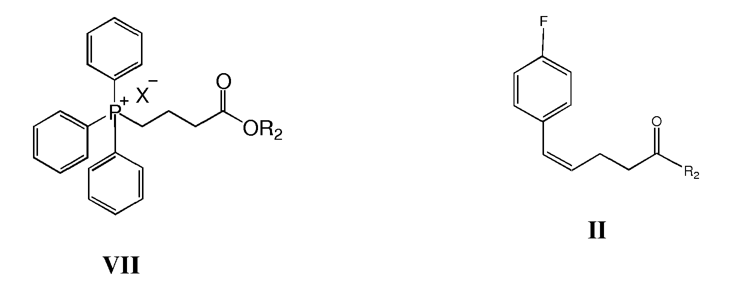 wherein R₂ is as defined in any one of (3) to (5).
   In this embodiment, using a suitable phosphonium-ylid compound which already bears a desired R₂ group obviates the need for proceeding through a compound of formula I.
   According to yet another aspect of the present invention, there is provided:
(13) A process for the synthesis of ezetimibe using any compound as defined by formulas I to V above. From any of these intermediate compounds, the skilled person can use conventional synthesis methods to finally yield ezetimibe.
   In a particular aspect of the present invention, there is provided:
(14) A process for the synthesis of ezetimibe comprising the steps of
   a) oxidizing a compound of formula V to obtain a ketone of formula IX shown below wherein R₄ is as defined above;
   b) reducing the ketone product of step (a) to obtain the corresponding (*S*)-hydroxy isomeric product, and
   c) subjecting the reduced (S)-hydroxy isomeric product to OH-deprotection reaction to obtain ezetimibe: 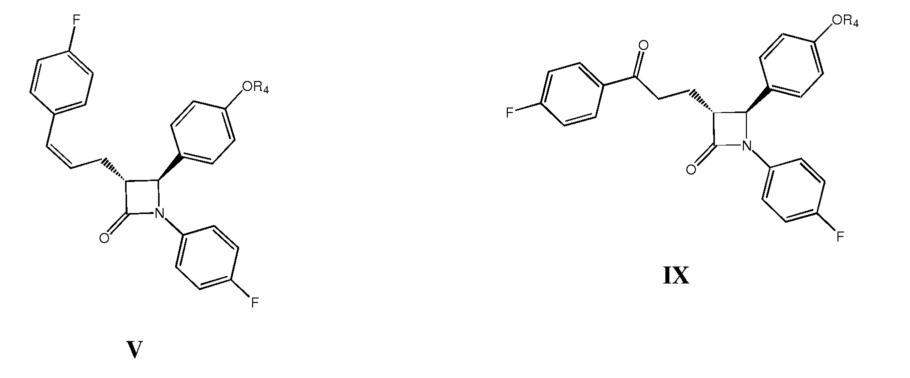 The present invention yet further provides:
(15) A process for the preparation of a pharmaceutical composition comprising ezetimibe as the active ingredient, comprising the steps of:
   preparing ezetimibe according to the process according to (13) or (14), and
   admixing the thus prepared ezetimibe with at least one pharmaceutically acceptable excipient.
(16) Ezetimibe essentially free of compound V and essentially free of the deprotected 4-hydroxyphenyl analog thereof.
(17) A process for the preparation of a pharmaceutical composition comprising ezetimibe as the active ingredient, comprising the steps of:
   a) providing ezetimibe essentially free of either or both of 3-Z- or -E-isomers of (3*R*,4*S*)-4-(4-(benzyloxy)phenyl)-1-(4-fluorophenyl)-3-((*Z*/*E*)-3-(4-fluorophenyl)-allyl)azetidin-2-one, and/or essentially free of the deprotected 4-hydroxyphenyl analogue thereof, and
   b) admixing the thus provided ezetimibe with at least one pharmaceutically acceptable excipient.
(18) A pharmaceutical composition comprising ezetimibe as the active ingredient and at least one pharmaceutically acceptable excipient, obtainable by the process according to (17).

### Brief description of the drawings

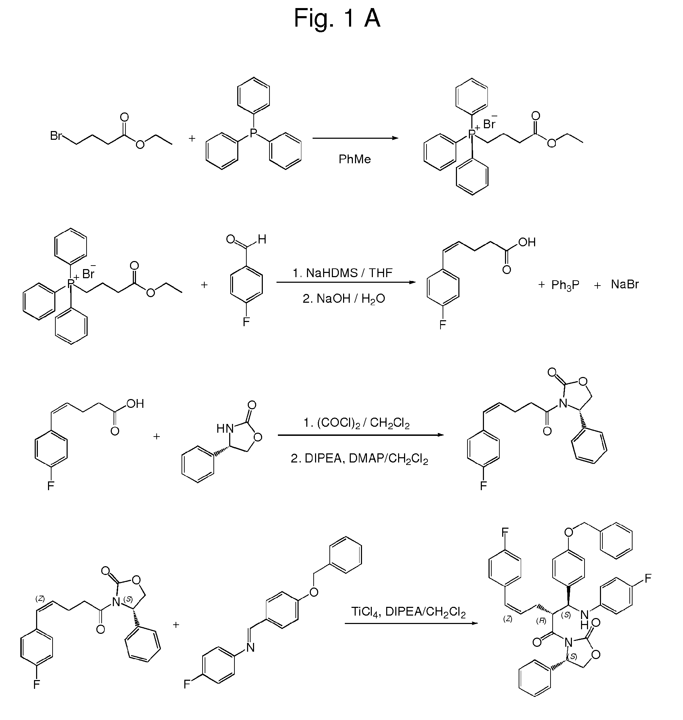

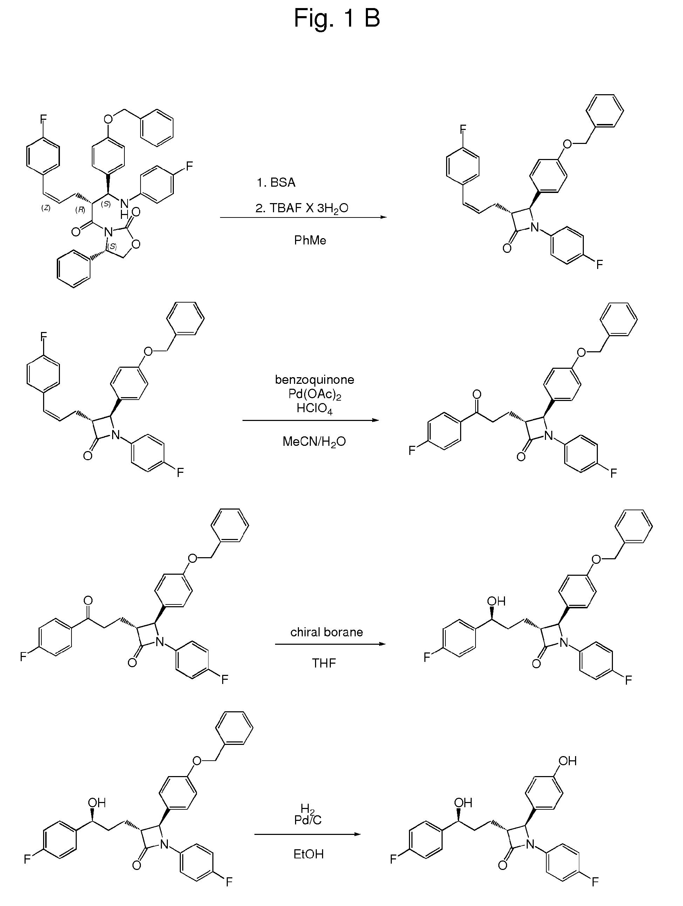

- Figs. 1A and 1B: schematically show a preferred synthesis route for the total synthesis of ezetimibe via exemplified *Z*-intermediates according to an embodiment of the present invention.

### Description of preferred embodiments

According to a particular aspect of the present invention, 5-(4-fluorophenyl)-pent-4-enoic acid and its derivatives as important intermediates for the total synthesis of ezetimibe are provided by a very effective synthetic approach via a Wittig reaction with high yields. In this process step, 4-fluoro-benzaldehyde is subjected together with a compound of formula VI to a Wittig reaction. Either the acid group with R₁=hydrogen is used, preferably the acid group is derivatized by R₁ being selected from respectively unsubstituted or substituted alkyl, cycloalkyl, alkenyl, cycloalkenyl, alkynyl, aryl, aralkyl, and arylcycloalkyl.

The obtained mixture of *E*- and *Z*-isomers of 5-(4-fluorophenyl)-pent-4-enoic acid or its derivatives can then be isolated as the *E*-isomer and the *Z*-isomer, respectively. Surprisingly, the Wittig reaction in the process according to the present invention not only significantly contributes to achieving high total yields, but more importantly generates predominantly the desired *Z*-isomer and thus allows to selectively isolate and obtain the representative key *Z*-intermediate for the synthesis of ezetimibe. Remarkably yields of over 90 % for the mixture of *Z*- and *E*-isomers and a range of about 80 % for the *Z*-isomer (ratio Z:E = 88:12) can specifically be achieved by using the Wittig reaction in embodiments of the present invention.

The desirable *Z*-isomer can be readily isolated and optionally purified from the *Z*- and *E-*isomer mixture, to thereby provide the compound of formula I:

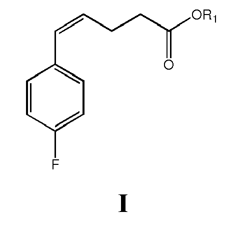

The residue R₁ is H or unsubstituted or substituted alkyl, unsubstituted or substituted cycloalkyl, unsubstituted or substituted alkenyl, unsubstituted or substituted cycloalkenyl, unsubstituted or substituted alkynyl, unsubstituted or substituted aryl, unsubstituted or substituted aralkyl, or unsubstituted or substituted arylcycloalkyl. The alkyl, alkenyl or alkynyl groups or the cyclic structures thereof in the aforementioned corresponding residues for R₁ may include organic groups containing 1 to 12, preferably 1 to 8 and more preferably 1 to 6 carbon atoms, without being limited thereto. A preferred residue R₁ is H or lower alkyl with 1 to 6 carbon atoms such as methyl, ethyl, propyl, isopropyl, n-butyl, tert-butyl, pentyl, hexyl, or the like. Suitable aryl groups may include phenyl, naphthyl, diphenyl, and the like, without being limited thereto. Suitable substituents may include lower alkyl with 1 to 6 carbon atoms, halogen, amine groups or the like, without being limited thereto.

According to a preferred embodiment, the Wittig reaction proceeds more efficiently when R₁ is selected from the above mentioned groups but is not H to obtain a corresponding 5-(4-fluorophenyl)pent-4-enoic acid derivative, notably an ester derivative, whereupon the obtained ester group can be subjected to saponification to give the corresponding 5-(4-fluorophenyl)-pent-4-enoic acid. The saponification is preferably performed as a one-pot proces after the Wittig reaction without isolation of an ester derivative. The compound of formula I, in particular wherein R₁ is H, can then be converted into the compound of formula II, wherein R₂ denotes the leaving group denoted above. According to a preferred embodiment, R₂ is an N-heterocyclic group, wherein the N-hetero atom forms an amido group with the carbonyl group in formula II. With a view to further enantioselective synthesis steps to give ezetimibe, it is more preferred that the N-heterocyclic group has a chiral centre and more preferably has a chiral S-isomeric centre. Thus a more preferred N-heterocyclic group is alkylated or arylated, for example by an alkyl group selected from a lower alkyl, such as methyl, ethyl, propyl, isopropyl, n-butyl, tert-butyl, or aryl, such as phenyl, naphthyl, either substituted or not, without being limited thereto.

Accordingly a compound with formula II can be provided in the desired *Z*-form, and wherein R₂ is defined above and is preferably defined by the structural moiety of formula III below, wherein alkyl and aryl is as defined above.

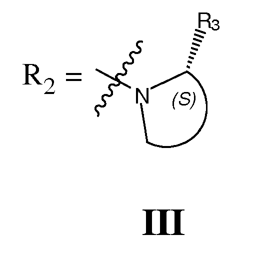

When according to an embodiment of the invention, the compound of formula II is obtained from the reaction with *Z*-5-(4-fluorophenyl)-pent-4-enoic acid, the acid group can be activated for the subsequent formation of the compounds of formula II, especially wherein the residue R₂ is defined by formula III. Activation of the acid group in the *Z*-5-(4-fluorophenyl)-pent-4-enoic acid can be carried out by known methods, for example via the formation of an acid halogenide such as acid chloride or acid bromide, as exemplified e.g. by a reaction respectively with phosgene, oxalyl chloride (COC)₂, thionyl chloride CISOCI, phosphorous pentachloride PCl₅, benzoyl bromide, or the like; via corresponding carbonic acid anhydrides.

For the above-mentioned Wittig reaction, an appropriate triphenyl phosphonium salt is prepared to obtain a compound of formula VI wherein X may be a halogen, such as Cl or Br, wherein R₁ is defined as above:

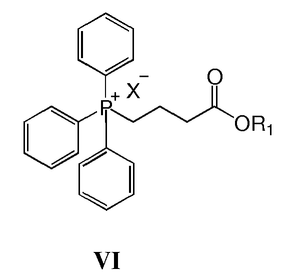

In a variation of a process according to the present invention, a compound of formula II can be directly obtained by carrying out a Wittig reaction of 4-fluoro-benzaldehyde with a corresponding compound of formula VII wherein R₂ is as defined above:

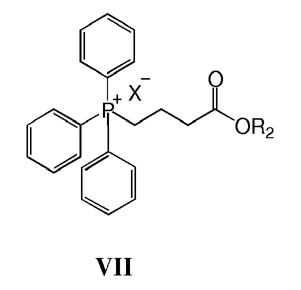

As the desirable *Z*-intermediate compound of formula II is used for the ezetimibe synthesis, the *Z*-isomeric form obtained from the Wittig reaction shall be isolated from the mixture of isomers.

In a subsequent step of the total synthesis route of ezetimibe, the compound of formula II, more preferably the one having the group R₂ as shown by formula III, is reacted with an imine compound defined by formula VIII shown below, wherein R₄ is an OH-protecting group including, but not limited to benzyl (Bn), benzyl carbonate (Cbz), benzyloxymethylether (BOM),; silyl, more preferably C₁ - C₈ trialkylsilyl, C₁ - C₈ dialkylarylsilyl, C₁ - C₈ alkyldiarylsilyl, wherein the alkyls may be the same or different, preferably aryl is phenyl and alkyls have 1 to 4 C-atoms; and the like, 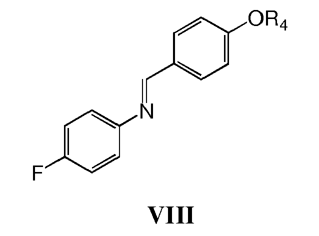 to give a compound with the formula IV, wherein R₂ and R₄ is as defined above

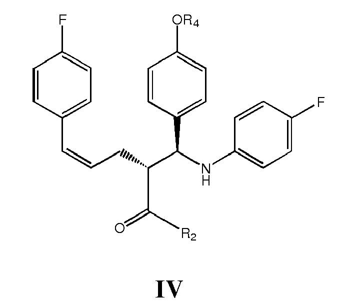

The compound IV can then be cyclized, suitably by a treatment with a silylating agent, such as bis-trimethylsilyl acetamide (BSA) and a subsequent treatment by a fluoride ion catalyst, such as tetrabutylammonium fluoride (TBAF), to thereby give a compound with the formula V wherein R₄ is as defined above:

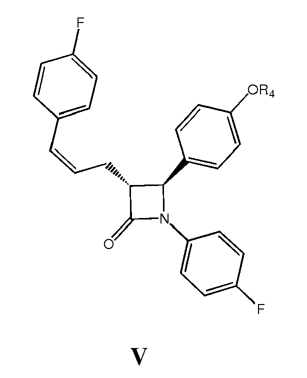

The compound of formula V having *Z*-isomeric structure then constitutes a useful intermediate for steps in the synthesis of ezetimibe wherein this compound is first oxidized to obtain the ketone of formula IX as defined above, then reducing the ketone to obtain and isolate the desired *S*-*OH*-isomeric product, for example by a reaction with a chiral borane, and finally subjecting the reduced *S*-*OH*-isomeric product to *OH*-deprotection reaction to finally yield ezetimibe. A suitable *OH*-deprotection reaction usually includes catalytic hydrogenation using metal catalysts such as Pt, Pd, Rh transition metals respectively alone or in admixture, preferably in combination with a support material, such as activated carbon, alumina, silica. The most preferred catalyst is palladium on carbon. As the hydrogen source to be present in the hydrogenation reaction, H₂ gas, ammonium formate / formic acid and the like may be used.

The process for the synthesis of ezetimibe according to the present invention is particularly advantageous in that the use of the *Z*-starting and intermediates compounds can provide unexpected high yields, referred to the total yield of ezetimibe, compared to the use of corresponding *E*-starting substances or intermediates. This is due to the design of the overall process (application of a "clean" Wittig reaction which gives a pure Z-acid compared to the Tetrahedron 2000, 56, pp. 5735-5742 where a complex mixture is obtained after the reaction) and the physical properties (solubility, chromatographic behaviour) of *Z*-starting and intermediate compounds which enable their isolation in a high purity and are therefore converted in each subsequent step with a lower extent of side reactions which would lower the overall yield. This effect becomes particularly pronounced in alkene V oxidation step and chiral borane reduction step of compound IX. Namely the oxidation of Z-alkene gives 86 % yield of ketone IX, while *E*-alkene oxidation gives ketone in 70-80 % yield as described in US-A-5 856 473. Moreover the ketone reduction step of the ketone derived through *Z-*intermediates gives 95 % yield while the ketone derived from *E*-intermediates gives only 42 % yield as described in US-A-5 856 473. The use of the compounds described above as intermediates for the process for the synthesis of ezetimibe therefore is a beneficial aspect of the present invention. Besides the special and preferred reactions described above, other conventional synthesis steps or known variations to finally yield ezetimibe - yet including the use of the presently disclosed *Z*-intermediate compounds - can be applied.

The ezetimibe prepared by the processes according to the present invention can then - usually after obtaining a desired purity level - be admixed with at least one pharmaceutical acceptable excipient. In order to obtain a pharmaceutically safe and stable pharmaceutical composition comprising ezetimibe, it is significant to make sure that ezetimibe is essentially free, preferably completely free of compound V before being admixed with any pharmaceutical excipient. The presence of compound V itself is considered to be detrimental to pharmacological characteristics and stability, in particular chemical stability, of the final pharmaceutical product, and moreover may be indicative of further detrimental impurities which might have been formed in the ezetimibe synthesis steps subsequent to the compound V intermediate. The significance of being impurity-free before being admixed with a pharmaceutical excipient resides in that the impurity could not be removed anymore after being co-processed into the final composition together with the active ezetimibe and the excipients, especially when solid dosage forms are contemplated. Without being bound to any theory, it may be believed that the double bond of the allyl group present in compound V may be critically involved in interfering reactions in the aforementioned final ezetimibe synthesis steps (for example in the enantiopure reduction to (*S*)-hydroxyl group at the 3-position of the compound [cf. step (b) under point (14) above] and/or of the final OH-deprotection reaction [cf. step (c) under point (14) above]) or in other final ezetimibe synthesis steps, and/or especially in the final co-admixture with excipients in the pharmaceutical composition containing ezetimibe. Essential freeness or even absence of compound V in the final ezetimibe product should be ensured by appropriate purification and isolation methods in or after the step when compound V is used as educt. With a view that not the totality of the compound V could be removed after the synthesis step where it was used as the educt, it is further relevant that the deprotected 4-hydroxyphenyl analog of compound V, which may eventually have been formed as another critical impurity in the final deprotection step for obtaining ezetimibe, is also surely removed before ezetimibe is admixed with pharmaceutical excipients in the preparation of the pharmaceutical composition.

The same observations would likewise apply if ezetimibe was synthesized through corresponding E-intermdiates. As the present invention is capable of proceeding through the Z-intermediates, it is however ensured that ezetimibe being admixed with any pharmaceutical excipient is also free of the corresponding E-isomer of (3*R*,4*S*)-4-(4-(benzyloxy)phenyl)-1-(4-fluorophenyl)-3-(*E*)-3-(4-fluorophenyl)-allyl)azetidin-2-one. Thus, the essential freeness and even total absence of the E-isomer is another merit of the invention to enable a pharmaceutically safe and stable ezetimibe-containing pharmaceutical composition.

As used herein, the terms "essentially free of compound V" (i.e. the Z-isomer) or correspondingly "essentially free of" the corresponding E-isomer of compound V or the respective deprotected 4-hydroxyphenyl (Z or E) analogs shall mean a control of its amount to below, for example, about 0.2 wt.-%, preferably less than about 0.1 wt.-%, more preferably less than about 0.05 wt.-% relative to the total weight of the yielded EZT product. Detection of compound V or its corresponding E-isomer or the respective deprotected 4-hydroxyphenyl analogs thereof, each with respect to the EZT product, can be carried out by HPLC. The term "free of" however may include the presence of at least trace amounts of the identified EZT impurities, indicative of whether EZT synthesis went through Z- or through E-intermediates.

The pharmaceutical composition can be used for the prevention and/or the treatment of any one of diseases selected form cholesteremic, hyperlipidemic and lipemic conditions, cancers and antibiotic conditions. A preferred use is hypercholesterolemia. A suitable dosage of ezetimibe may be in a range of 0.1 mg to 100 mg, more suitably from 1 mg to 20 mg. The pharmaceutically acceptable excipient can be chosen from suitable carriers and excipients known to a person skilled in the art. For example, suitable pharmaceutically acceptable carriers and/or excipients may include, but are not limited to binders, fillers, matrix forming agents, diluents, antioxidants, buffering agents, antifoaming agents, preservatives, chelating agents, viscomodulators, flavourants, colourants, odourants, opacifiers, stabilizing agents, solubilizers, plasticizing agents, lubricants, and mixtures thereof. Suitable specific examples include, but are not limited to those of the group consisting of lactose, microcrystalline cellulose, cellulose derivatives such as hydroxypropylcellulose, polyacrylates, calcium carbonate, starch, colloidal silicone dioxide, sodium starch glycolate, talc, magnesium stearate, polyvinylpyrrolidone, polyethylene glycol and other excipients known in the field of the pharmaceutical technology.

The excipients may also be chosen depending on the desired administration and dosage form. Suitable administration forms include oral, parenteral, injectable, transdermal, nasal, rectal, inhalable, without being limited thereto. Suitable dosage forms include tablets, capsules, granules, powders, pellets, suspensions, liquids, suppositories, sustained release dosage forms, without being limited thereto. In the pharmaceutical preparation according to the present invention, ezetimibe may also be combined with one or more other active ingredients.

The present invention is described in further detail below by referring to examples, which however are provided only for illustrative purposes and the invention is in no way limited thereto.

A particularly preferred synthesis route for the total synthesis of ezetimibe according to preferred reaction steps is shown in Figs. 1A and 1B. Relevant reaction steps are described in more detail in the examples below.

### Examples

### Example 1: Synthesis of (4-ethoxy-4-oxobutyl)triphenylphosphonium bromide (1)

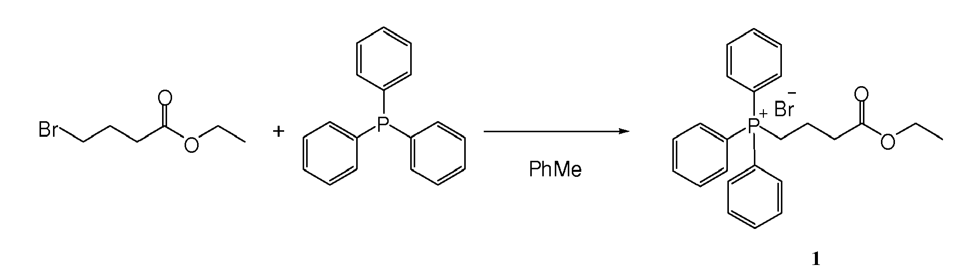

A solution of ethyl 4-bromobutyrate (51.0 g, 261 mmol) and triphenylphosphine (67.2 g, 256 mmol) in dry toluene (250 mL) was stirred and heated under reflux and under argon atmosphere for 4 days. After cooling the reaction mixture to room temperature the formed insoluble white precipitate was filtered off and washed with hot toluene (100 mL) and hexane (80 mL). The product was pulverized and dried under the reduced pressure to give the title compound **1** (113.7 g, 97 %) as white powder.

### Example 2: Synthesis of (Z)-5-(4-fluorophenyl)pent-4-enoic acid (3)

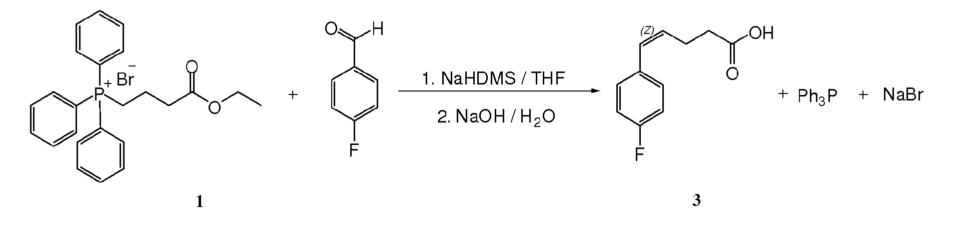

Powdered (4-ethoxy-4-oxobuthyl)triphenylphosphonium bromide (**1**) (10.5 g, 23.7 mmol) in dry THF (30 mL), under argon atmosphere, was treated with sodium hexamethyldisilazide (12.5 mL, 25 mmol; 2 M solution in THF) at 18 °C with stirring. After 20 min, 4-fluorobenzaldehyde (2.61 g, 21.0 mmol) was added at 18 °C, rapidly decolourizing the red-orange mixture. After stirring at room temperature for 4 hours, the reaction mixture was cooled down on an ice bath and NaOH (1.6 g, 40 mmol) in 10 mL of water was added. The resulting solution was stirred at room temperature for additional 12 hours. Water (100 mL) and ether (200 mL) were added. The phases were separated and the water phase was rinsed with ether (2×50 mL), acidified (4 M HCl) and extracted with EtOAc (4×50 mL). The collected EtOAc extracts were dried with anhydrous Na₂SO₄, and concentrated. Kugelrohr distillation gave 3.52 g (90.6 %) of pure 5-(4-fluorophenyl)pent-4-enoic acid (**3**) as white crystals, containing 88 % of cis isomer which can be obtained in a pure form after recrystallization of an ester derivative, an acid salt derivative followed by acidification or a crude acid.
Compound **2** (cis- ester):
   ¹H-NMR (300 MHz, CDCl₃): δ (ppm)= 1.26 (t, 3H, *J* = 7.1 Hz, -CH₃), 2.42-2.47 (m, 2H, CH₂CH₂CO), 2.60-2.68 (m, 2H, CH₂CH₂CO), 4.15 (q, 2H, *J* = 7.1 Hz, -CH₂-CH₃), 5.63 (td, 1 H, *J* = 7.2 Hz, *J* = 11.6 Hz, CH=CHCH₂), 6.44 (d, 1 H, *J* = 11.6 Hz, PhCH=CH), 7.00-7.08 (m, 2H, Ph-H), 7.22-7.28 (m, 2H, Ph-H) ppm.
Compound **3** (cis- carboxylic acid):
   ¹H-NMR (300 MHz, CDCl₃): δ (ppm)= 2.46-2.51 (m, 2H, CH₂CH₂CO); 2.59-2.66 (m, 2H, CH₂CH₂CO), 5.64 (td, 1H, *J* = 7.0 Hz, *J* = 11.6 Hz, CH=CHCH₂), 6.44 (d, 1H, *J* = 11.6 Hz, PhCH=CH), 7.00-7.08 (m, 2H, Ph-H), 7.22-7.27 (m, 2H, Ph-H), 9.99 (bs, 1H, -COOH) ppm.

### Example 3:

### Synthesis of (S,Z)-3-(5-(4-fluorophenyl)pent-4-enoyl)-4-phenyloxazolidin-2-one (4)

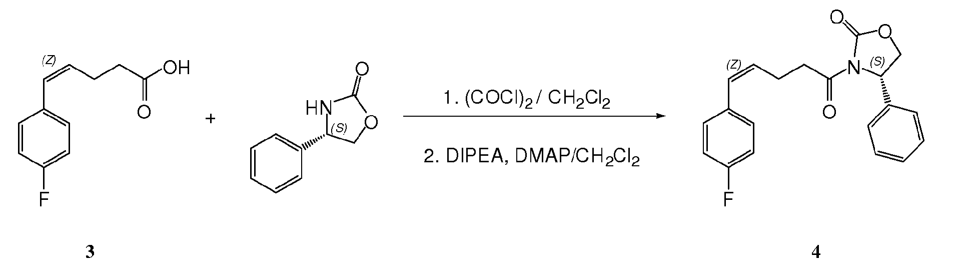

To a solution of p-fluorophenyl pentenoic acid (**3**) (2.3 g) in CH₂Cl₂ (40 mL) oxalyl chloride (1.81 g) and DMF (2 drops) were slowly added at 0 °C. The solution was warmed to room temperature and refluxed for 1 h. The solvent was evaporated under the reduced pressure and the excess of oxalyl chloride was removed azeotropicly with CH₂Cl₂ (2x50 mL). The resultant acid chloride was redissolved in CH₂Cl₂ (40 mL) and the solution was added dropwise to a cooled solution of (*S*)(+)-4-phenyl-2-oxazolidone (1.99 g), *N*,*N-*diisopropylethylamine (DIPEA) (3.07 g) and *N*,*N*-dimethylaminopyridine (0.05 g) in CH₂Cl₂ (20 mL). The resulting mixture was stirred at room temperature for 2 hours. After CH₂Cl₂ (100 mL) was added and the organic layer washed with 0.5 M HCl (2×40 mL), water (1 x40 mL) and brine (1×40 mL), then dried and concentrated to crystallize the desired product. The product was recrystallized from ethyl acetate / hexane and collected by filtration (3.7 g, 93 %).

¹H-NMR: (300 MHz, CDCl₃): δ 2.55-2.70 (m, 2H, CH₂CH₂CO), 3.05-3.15 (m, 2H, CH₂CH₂CO), 4.29 (dd, J = 8.91 Hz, J = 3.69 Hz, 1 H, CHCH₂OCO), 4.69 (t, J = 8.82 Hz, 1 H, CHCH₂OCO), 5,43 (dd, J = 8.71 Hz, J = 3.66 Hz, 1 H, CHCH₂OCO), 5.57-5.66 (m, 1 H, ArCH=CH), 6.40 (d, J = 11.56 Hz, 1 H, ArCH=CH), 6.95-7.04 (m, 2H, ArH), 7.20-7.43 (m, 7H, ArH) ppm.

### Example 4: Synthesis of (S)-3-((R,Z)-2-((S)-(4-(benzyloxy)phenyl)(4-fluorophenyl amino)methyl)-5-(4-fluorophenyl)pent-4-enoyl)-4-phenyloxazolidin-2-one (6)

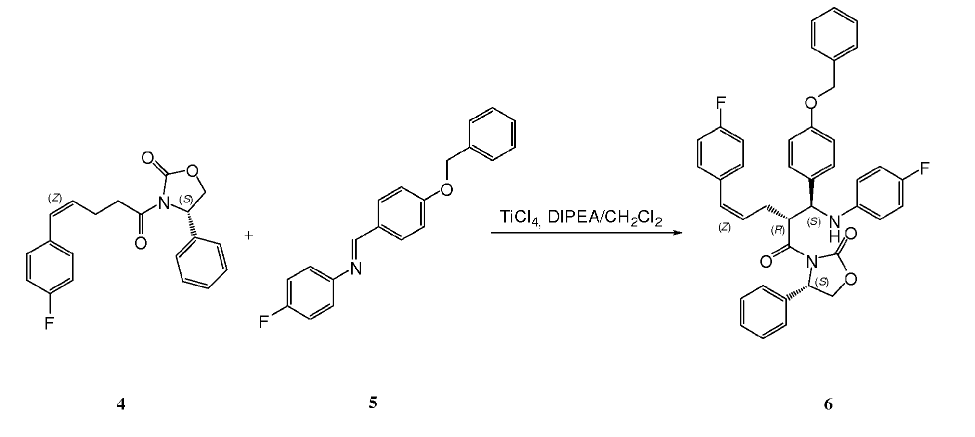

To a solution of (*S*,*Z*)-3-(5-(4-fluorophenyl)pent-4-enoyl)-4-phenyloxazolidin-2-one (**4**) (1.0 g) in CH₂Cl₂ (15 mL) at -20 °C, 1 M solution of TiCl₄ in CH₂Cl₂ (3,25 mL) was slowly added and stirred at -20 °C. After 15 min, DIPEA (0.97 mL) was added and the mixture was stirred for 30 min at -20 °C. (*E*)-*N*-(4-(benzyloxy)benzylidene)-4-fluoroaniline (**5**) (1.53 g) was dissolved in CH₂Cl₂ (30 mL) and added to the solution while temperature was kept below -20 °C. After 1.5 h, the reaction was quenched with glacial acetic acid (1 mL) in CH₂Cl₂ (3 mL) at -20 °C and stirred for 30 min. The reaction mixture was poured into aqueous solution of 1 M H₂SO₄ (40 mL) and mixed for another 30 min. After that, 150 mL of ethyl acetate were added and the organic layer washed with the saturated solution of NaHCO₃ (3x40 mL) and brine (1 x40 mL), then dried with Na₂SO₄ and concentrated. The product was recrystallized from ethyl acetate / hexane and collected by filtration. The crystals (1.15 g; 59 %) were washed with cold methanol.

¹H-NMR: (300 MHz, CDCl₃): δ 2.31-2.40 (m, 1H, =CHCH₂CH), 2.65-2.75 (m, 1H, =CHCH₂CH), 4.22 (dd, J = 8.76 Hz, J = 3.13 Hz, 1 H, CHCH₂OCO), 4.39 (t, J = 9.42 Hz, 1H, =CHCH₂CH) 4.36-4.60 (m, 1 H, CHNH), 4.69 (t, J = 8.67 Hz, 1 H, CHCH₂OCO), 4.95 (d, J = 10.32 Hz, 1H, NH), 5.03 (s, 2H, CH₂Ph), 5.43 (dd, J = 8.45 Hz, J = 3.11 Hz, 1H, CHCH₂OCO), 5.51-5.60 (m, 1 H, ArCH=CH), 6.24-6.42 (m, 1 H, ArCH=CH), 6.69-6.81 (m, 2H, ArH), 6.84-6.96 (m, 4H, ArH), 7.08-7.18 (m, 10H, ArH), 7.38-7.50 (m, 7H, ArH) ppm.
MS (ES+) m/z (%): 645 (MH+, 60), 534 (100).

### Example 5:Synthesis of (3R,4S)-4-(4-(benzyloxy)phenyl)-1-(4-fluorophenyl)-3-((Z)-3-(4-fluorophenyl)-allyl)azetidin-2-one (7)

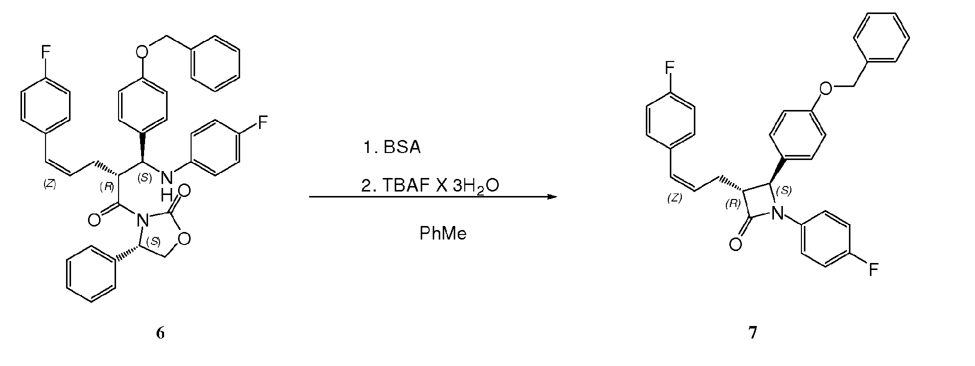

A suspension of **6** (0.484 g, 0.75 mmol) in dry toluene (5.0 mL) was deoxygenated with argon and after 15 minutes, BSA (1.50 mmoL, 0.37 ml) was added. After 30 minutes of stirring at room temperature, TBAFx3H₂O (5.0 mol %, 0.0375 mmol, 11.8 mg) was added and left stirring for 4 hours. Then 0.027 ml of glacial acetic acid and 4.0 ml of MeOH was added. After 5 minutes, the reaction mixture was concentrated under the reduced pressure and EtOAc (50 ml) was added. The organic layer was washed with 5 % sodium bicarbonate solution (25 ml), water (25 ml) and brine (25ml), dried with sodium sulfate, filtered and concentrated under the reduced pressure. The crude product was purified by flash chromatography using EtOAc/hexane (1/4) as the eluent to obtain 0.256 g (71 %) of compound 7.

¹H-NMR (300 MHz, CDCl₃): δ (ppm)= 2.74-2.95 (m, 1H, CH₂), 3.13-3.19 (m, 1H, CHCO), 4.52 (d, *J*= 2.3 Hz, 1 H, CHN), 5.07 (s, 2H, OCH₂Ph), 5.63-5.71 (m, 1 H, PhCHCH), 6.52 (d, *J* =11.5 Hz, 1 H, PhCHCH), 6.86-7.04 (m, 6H, Ph-H), 7.15-7.24 (m, 6H, Ph-H), 7.30-7.41 (m, 5H, Ph-H).

### Example 6: Synthesis of (3R,4S)-4-(4-(benzyloxy)phenyl)-1-(4-fluorophenyl)-3-(3-(4-fluorophenyl)-3-oxopropyl)azetidin-2-one (8)

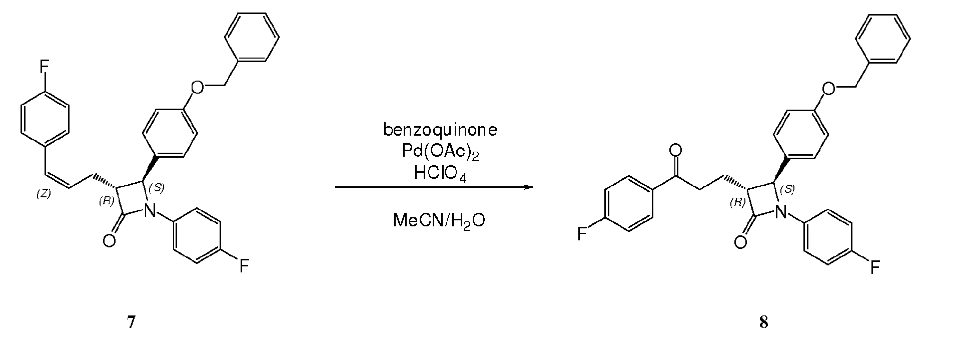

Pd(OAc)₂ (3.0 mol %, 0.042 mmol, 9.43 mg), benzoquinone (2.1 mmol, 227 mg) and perchloric acid (70 % aqueous solution, 0.15 M, 0.100 ml) were dissolved in acetonitrile (3.5 mL) and deoxygenated by purging with argon for at least 20 minutes. Water (0.7 ml), which was also deoxygenated with argon, was then added. The reaction mixture was stirred vigorously for another 5 minutes under argon atmosphere and the solution of **7** (1.40 mmol) in acetonitrile (3.5 ml), which was previously also deoxygenated by purging with argon for 30 minutes, was added. After 4 hours, perchloric acid (70 % aqueous solution, 0.100 ml) was again added. The resulting solution was stirred for 48 hours and then diluted with EtOAc (50 ml). The water layer was washed with another 30 ml of EtOAc. Organic layers were combined and washed with water (2x40 m) and brine (40 ml), dried with sodium sulfate, filtered and concentrated under the reduced pressure. The crude product was purified by flash chromatography, eluted with EtOAc/hexane (1/4) to obtain the compound **8** (0,600 g) in 86 % yield.

¹H-NMR (300 MHz, CDCl₃): δ (ppm)= 2.21-2.47 (m, 2H, CH₂CH), 3.10-3.21 (m, 2H, CH₂CO), 3.24-3.35 (m, 1H, CHCO), 4.68 (d, *J* =2,3 Hz, 1 H, CHN), 5.05 (s, 2H, OCH₂Ph), 6.90-6.99 (m, 4H, Ph-H), 7.09-7.17 (m, 2H, Ph-H), 7.22-7.27 (m, 4H, Ph-H), 7.33-7.44 (m, 5H, Ph-H), 7.96-8.02 (m, 2H, Ph-H).

### Example 7: Synthesis of (3R,4S)-4-(4-(benzyloxy)phenyl)-1-(4-fluorophenyl)-3-((S)-3-(4-fluorophenyl)-3-hydroxypropyl)azetidin-2-one (9)

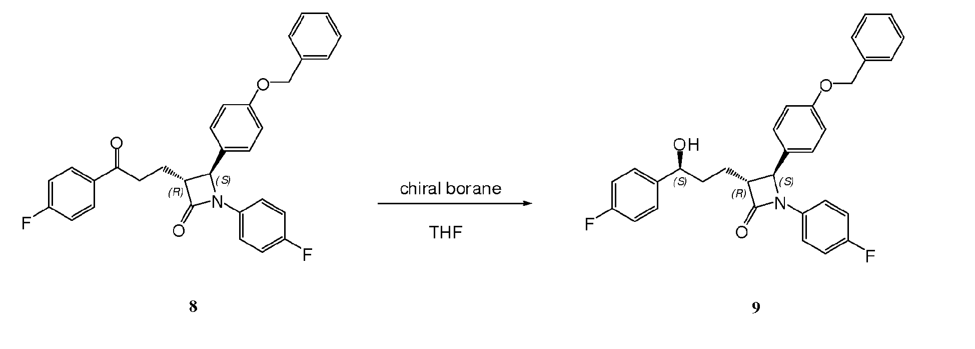

(*R*)-tetrahydro-1-methyl-3,3-diphenyl-1*H*,3*H*-pyrrolo[1,2-c]-[1,3,2]oxazaborole (96 mg; 0.346 mmol) and (3*R*,4*S*)-4-(4-(benzyloxy)phenyl)-1-(4-fluorophenyl)-3-(3-(4-fluorophenyl)-3-oxopropyl)azetidin-2-one (**8**) (760 mg; 1.528 mmol) were dissolved in dry THF (2 mL) under argon atmosphere. The solution was cooled to -22 ºC, and after stirring for 5 minutes, borohydride-dimethylsulfide complex (2 M solution in THF; 0.864 mL; 1.728 mmol) was added dropwise over 2 hours. After stirring a total of 5 h at -22 ºC, the reaction was quenched by the addition of methanol (3 mL). EtOAc (30 mL) and 1 M HCl (15 mL) were added and the phases were separated. The water phase was extracted twice with EtOAc (20 mL). The combined EtOAc extracts were dried with anhydrous Na₂SO₄, and concentrated to stable foam (0.900 g). The resulting crude product was purified by flash chromatography using EtOAc/hexane = 1/3 as a mobile phase to obtain the title compound **9** (0.731 g, 95.8 %).

¹H-NMR (300 MHz, CDCl₃): δ (ppm)= 1.84-2.05 (m, 4H; CH₂CH₂); 3.07-3.17 (m, 1H, CHCO); 4.59 (d, *J* = 2.4 Hz; 1H, CHN); 4.71-4.76 (m, 1H, CHOH); 5.07 (s, 2H, OCH₂Ph); 6.91-7.06 (m, 6H, Ph-H); 7.22-7.46 (m, 11 H, Ph-H) ppm

### Example 8: Synthesis of ezetimibe (10)

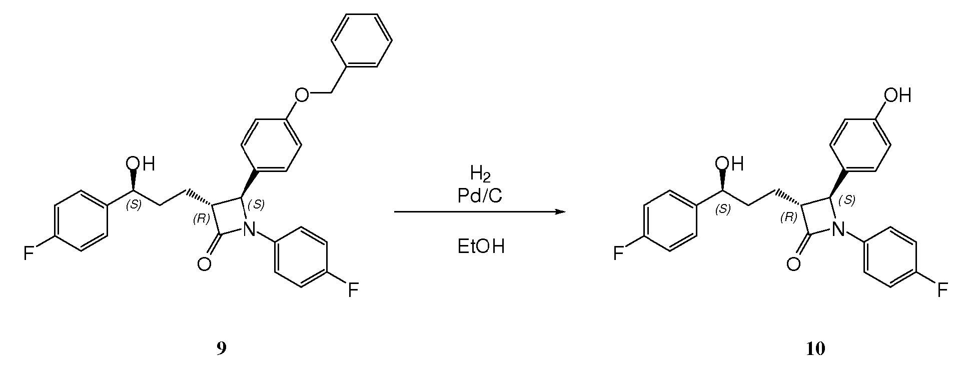

(3*R*,4*S*)-4-(4-(benzyloxy)phenyl)-1-(4-fluorophenyl)-3-((*S*)-3-(4-fluorophenyl)-3-hydroxypropyl)azetidin-2-one **(9)** was dissolved in ethanol and 5 % Pd/C (0.5 mol %) was added to the obtained solution. The obtained suspension was stirred under H₂ gas atmosphere at the elevated pressure until all the starting material **9** has been consumed. The catalyst was removed by filtration. The obtained solution was concentrated under the reduced pressure and recrystallized to afford the title compound **10.**

### Results:

The data on yields for individual steps and on the overall yield of the total synthesis of ezetimibe are presented in Tables 1 and 2 below, separated in results according to the present invention based on *Z*-intermediates and that according to prior art based on *E-*intermediates, respectively.

**Table 1: Overall yields of the synthesis of ezetimibe via Z-intermediates.**

| Yield per step [%] | | | | | | | | |
|---|---|---|---|---|---|---|---|---|
| Synthetic step | 1 | 2 | 3 | 4 | 5 | 6 | 7 | 8 |
| | Acid synthesis | | Ezetimibe synthesis | | | | | |
| | | | *Z*-ezetimibe intermediates | | | | Reduction | deprot. |
| *Z*-acid and intermediates | 97 | 80 | 93 | 59 | 71 | 86 | 95 | no data |
| | 77.6 | | 33.5 | | | | 95 | no data |
| | 77.6 | | 31.8 | | | | | no data |
| present invention | 24.7 | | | | | | | no data |
| | | | | | | | | |

**Table 2: Overall yields of the synthesis of ezetimibe via E-intermediates as reported in US 5,856,473.**

| Yield per step [%] | | | | | | | |
|---|---|---|---|---|---|---|---|
| Synthetic step | 1 | 2 | 3 | 4 | 5 | 6 | 7 |
| | Acid synthesis | Ezetimibe synthesis | | | | | |
| | | *E*-ezetimibe intermediates | | | | reduction | deprot. |
| *E*-acid and intermediates | 56 | 95 | 67 | 90 | 70-80 | 42 | no data |
| | | 40.1 - 45.8 | | | | 42 | no data |
| Data from US5,856,473 and Tetrahedron 56 (2000) 5735 - 5742 | 56 | 16.8 - 19.2 | | | | | no data |
| | 9.4 - 10.8 | | | | | | no data |
| | | | | | | | |

**Table 3: Overall yields of synthesis of ezetimibe via E-intermediates by procedures described in US 5,856,473 performed by our hands.**

| Yield per step [%] | | | | | | | |
|---|---|---|---|---|---|---|---|
| Synthetic step | 1 | 2 | 3 | 4 | 5 | 6 | 7 |
| | Acid synthesis | Ezetimibe synthesis | | | | | |
| | | *E*-ezetimibe intermediates | | | | reduction | deprot. |
| *E*-acid and intermediates | 5^{a}-17^{b} | 79 | 75 | 20 | 36 | 34 | no data |
| | | 4.3 | | | | 34 | no data |
| Data from US5,856,473 *Tetrahedron 56* (2000) 5735 - 5742 | 5-17 | 1.5 | | | | | no data |
| | 0.07 - 0.25 | | | | | | no data |
| | ^{a} the yield obtained by the exact repetition of the *Tetrahedron* procedure ^{b} the yield obtained by the reaction under Ar-atmosphere | | | | | | |

Overall yield for the steps disclosed in US 5,856,473 with *E*-derivatives: 16.8 % - 19.2 %.
Overall yield for the process in US 5,856,473 with *E*-derivatives in our hands: 1.5 %
Overall yield for the same steps obtained by the present invention with *Z*-derivatives: 31.8 %
Overall yield combining Tetrahedron 56 (2000) 5735 - 5742 and US 5,856,473: 9.4 % - 10.7 %
Overall yield combining Tetrahedron 56 (2000) 5735 - 5742 and US 5,856,473 in our hands: 0.07 % - 0.25 %
Overall yield obtained by the present invention with *Z*-derivatives: 24.7 %

## Claims

1. A compound with the following formula I 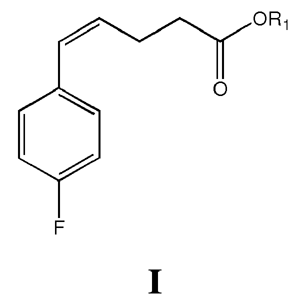 wherein R₁ is H or, respectively unsubstituted or substituted organic group selected from alkyl, cycloalkyl, alkenyl, cycloalkenyl, alkynyl, aryl, aralkyl and arylcyloalkyl.

2. The compound according to claim 1 wherein R₁ is H.

3. A compound with the following formula II 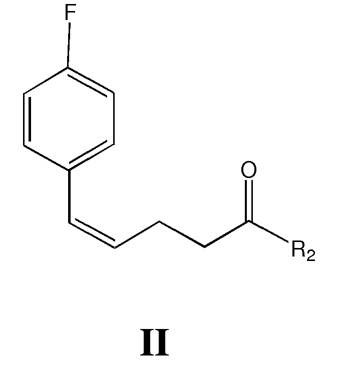 wherein R₂ is a leaving group.

4. The compound according to claim 3 wherein R₂ is an N-heterocyclic group, the N-heteroatom forming an amido group with the carbonyl group in formula II, preferably wherein R₂ is defined by the following formula III:

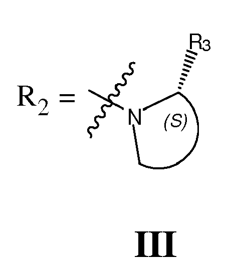

5. A compound with the following formula IV 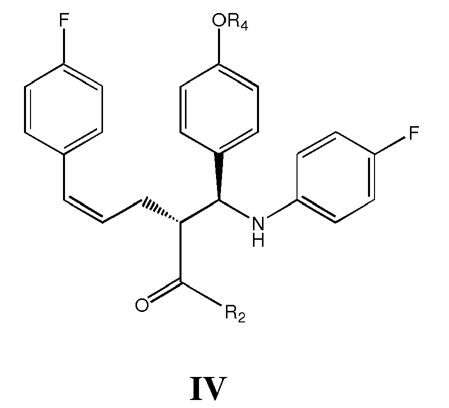 wherein R₂ is as defined in claim 3, and R₄ is an OH-protecting group.

6. A compound with the following formula V 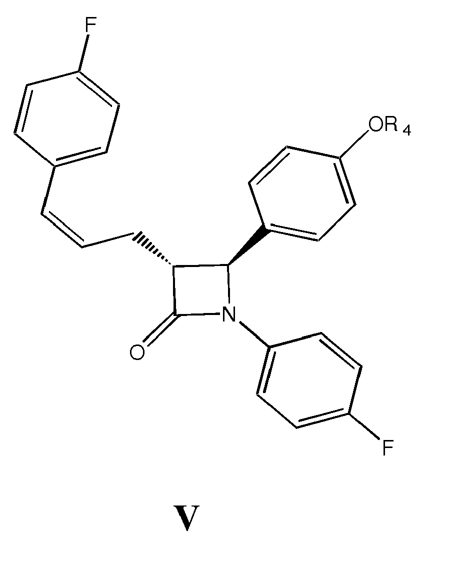 wherein R₄ is an OH-protecting group.

7. A process for producing 5-(4-fluorophenyl)pent-4-enoic acid or a derivative thereof comprising subjecting 4-fluoro-benzaldehyde and a compound of formula VI to a Wittig reaction: 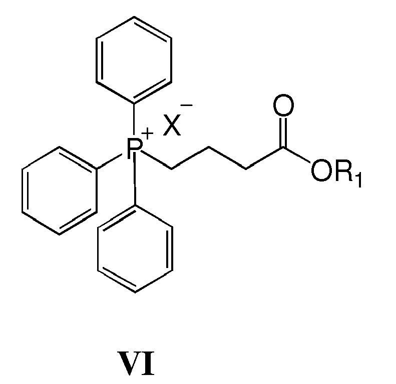 wherein X⁻ is a phosphonium salt anion, preferably halogenide, and R₁ is as defined in claim 1.

8. The process according to claim 7, wherein the (E,Z) isomers of the obtained 5-(4-fluorophenyl)pent-4-enoic acid derivatives are isolated as the E-isomer and the Z-isomer, respectively.

9. The process according to claim 7 or 8, wherein the isolated isomer is the Z-isomer compound with the following formula I 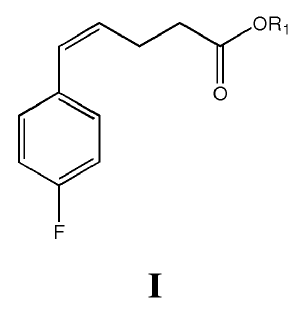 wherein R₁ is as defined in claim 1.

10. The process according to any one of claims 7 to 9 wherein an ester derivative is used as the compound of formula VI, and after the Wittig reaction the obtained ester group is subjected to saponification to give the corresponding 5-(4-fluorophenyl)-pent-4-enoic acid.

11. A process for producing a compound of formula II by a Wittig reaction of 4-fluoro-benzaldehyde with a compound of formula VII 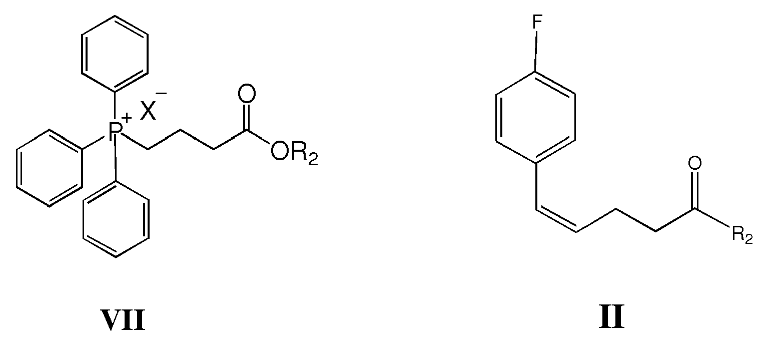 wherein X- is a phosphonium salt anion, preferably halogenide, and R₂ is as defined in claim 3 or 4.

12. A process for the synthesis of ezetimibe using any compound as defined in claims 1 to 6 as intermediates.

13. A process for the synthesis of ezetimibe comprising the steps of
a) oxidizing the compound of formula V to obtain a ketone of formula IX shown below wherein R₄ are as defined above;
b) reducing the ketone product of step (a) to obtain (S)-OH isomeric product, and
c) subjecting the reduced (S)-OH product to OH-deprotection reaction to obtain ezetimibe:

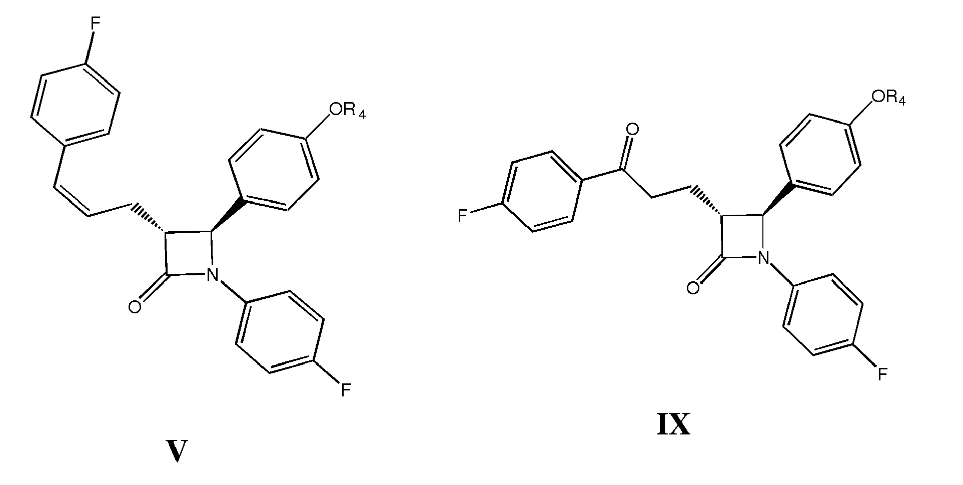

14. A process for the preparation of a pharmaceutical composition comprising ezetimibe as an active ingredient, comprising the steps of:
a) preparing ezetimibe according to the process according to claim 12 or claim 13, and
b) admixing thus prepared ezetimibe with at least one pharmaceutically acceptable excipient.

15. Ezetimibe essentially free of compound V and essentially free of the deprotected 4-hydroxyphenyl analog thereof.

16. A process for the preparation of a pharmaceutical composition comprising ezetimibe as the active ingredient, comprising the steps of:
a) providing ezetimibe essentially free of either or both of 3-Z- or -E-isomer of (3*R*,4*S*)-4-(4-(benzyloxy)phenyl)-1-(4-fluorophenyl)-3-((*Z*/*E*)-3-(4-fluorophenyl)-allyl)azetidin-2-one, or essentially free of the deprotected 4-hydroxyphenyl analogue thereof, and
b) admixing thus provided ezetimibe with at least one pharmaceutically acceptable excipient.

17. A pharmaceutical composition comprising ezetimibe as the active ingredient and at least one pharmaceutically acceptable excipient, obtainable by the process according to claim 16.
